# EUROPEAN PATENT SPECIFICATION

(11) **EP 4 072 417 B1**
(45) Date of publication and mention of the grant of the patent: **28.01.2026**
(21) Application number: 20898007.8
(22) Date of filing: 11.12.2020
(51) Int. Cl.: A61B 5/145, A61B 5/15, G01N 33/68, G01N 27/327, C12Q 1/68, C12N 15/11, A61B 5/1486, A61B 5/00, A61B 5/1468, C12Q 1/00, G01N 33/543

(54) **DEVICES AND METHODS FOR APTAMER-ASSISTED MICRONEEDLE-BASED MONITORING OF BIOMARKERS**
VORRICHTUNGEN UND VERFAHREN ZUR APTAMER-UNTERSTÜTZTEN MIKRONADELBASIERTEN ÜBERWACHUNG VON BIOMARKERN
DISPOSITIFS ET PROCÉDÉS PERMETTANT LA SURVEILLANCE DE BIOMARQUEURS, BASÉE SUR DES MICRO-AIGUILLES ASSISTÉE PAR DES APTAMÈRES

(30) Priority: 12.12.2019 US 201962947399 P
(43) Date of publication of application: 19.10.2022
(60) Divisional application: 25226542.6
(73) Proprietor: The Regents of the University of California, Oakland, CA 94607-5200 (US); The Johns Hopkins University, Baltimore, Maryland 21218 (US); President and Fellows of Harvard College, Cambridge, MA 02138 (US)
(72) Inventor: WANG, Joseph, San Diego, California 92101 (US); TEYMOURIAN, Hazhir, La Jolla, California 92093 (US); ARROYO, Netz, La Jolla, California 92093 (US); DASSAU, Eyal, La Jolla, California 92093 (US); TEHRANI, Farshad, La Jolla, California 92093 (US)
(74) Representative: Potter Clarkson
(86) International application number: PCT/US2020/064700
(87) International publication number: WO 2021/119546

(56) References cited:
- US-A1- 2003 068 666
- US-A1- 2010 022 416
- US-A1- 2011 210 017
- US-A1- 2013 225 956
- US-A1- 2017 055 835
- US-A1- 2018 279 929
- US-A1- 2018 340 203
- US-A1- 2019 101 551
- US-A1- 2019 125 223
- US-A1- 2019 223 795
- US-A9- 2005 101 841
- GAO JIE ET AL: "Simultaneous detection of glucose, uric acid and cholesterol using flexible microneedle electrode array-based biosensor and multi-channel portable electrochemical analyzer", SENSORS AND ACTUATORS B: CHEMICAL, vol. 287, 6 February 2019 (2019-02-06), NL, pages 102 - 110, XP093206047, ISSN: 0925-4005, DOI: 10.1016/j.snb.2019.02.020

## Description

### CROSS-REFERENCE TO RELATED APPLICATIONS

This application claims priority to the provisional application with serial number 62/947,399, titled "DEVICES AND METHODS FOR APTAMER-ASSISTED MICRONEEDLE-BASED MONITORING OF PROTEIN BIOMARKERS", filed on December 12, 2019.

### TECHNICAL FIELD

The subject matter of this patent document relates generally to electrochemical sensing, and in particular to methods, systems, materials and devices for label-free, continuous real-time monitoring of biomarkers in a biofluid.

### BACKGROUND

Currently there is no available device for the minimally-invasive or non-invasive continuous real-time monitoring of, for example, protein biomarkers in the interstitial fluid (ISF) and the only methods available for measuring levels of these highly important diagnostic markers are based on the traditional way of sampling blood using hypodermic needles and following quantification of the biomarkers using external meters/techniques in centralized laboratories. The ELISA method typically used for biomarkers quantification is highly time-consuming and expensive. Traditional approaches to biomarkers quantification have significant disadvantages, including, for example, patient discomfort, the need for a relatively high sample volume, and the generation of sharp waste. More importantly, they cannot track the trends and fluctuations in the levels of biomarkers in real time and thus, cannot provide real-time information regarding the concentrations of the biomarkers. Accordingly, there is still a need to produce low-cost systems, devices, materials and methods for continuous real-time monitoring of biomarker levels in the ISF.

US 2013/225956 A1 discloses a transdermal sensor for detecting a concentration of a hypodermal target molecule, comprising: a substrate; a plurality of microneedles fixed on said substrate; a signal processing unit, which is electrically connected to said microneedles; and a power supply unit for providing the working power. The transdermal sensor of the present invention detects long-term, real-time concentration of a hypodermal target molecule for a doctor to evaluate a physiological status of an user with minimal invasive piercing and pain.

US 2019/223795 A1 discloses a system for monitoring at least presence of a bioanalyte. The system comprises: a substrate having a skin contact surface, a microneedle outwardly protruding from the skin contact surface, a sensing complex positioned in the microneedle, and a circuit attached to the substrate. The sensing complex can comprise a nanostructure modified by a functional moiety covalently attached thereto, and an affinity moiety effective to react specifically with the bioanalyte to produce a reaction product. The circuit can apply voltage to the nanostructure and monitor changes in an electrical property of the nanostructure responsively to reaction between the reaction product and the functional moiety.

US 2018/279929 A1 discloses a transdermal microneedle array patch, including: a bottom cover; a top cover; a substrate disposed within the top cover; and a first probe and a second probe disposed between the bottom cover and the top cover and electrically connected the substrate. The first and second probes form an open circuit. While the bottom cover is combined with the top cover to form the transdermal microneedle array patch, the first and second probes form a closed circuit.

US 2019/101551 A1 discloses implantable sensors that can be deployed to the circulatory system of an animal where they can accurately and continuously measure the concentration of a target species, such as a drug, with very short resolution times, for extended periods without signal drift. These sensor designs and associated methods provide a means of accurately dosing animals based on real-time monitoring of drugs and other chemical markers and biomarkers.

US 2019/125223 A1 discloses methods, structures, and systems for biosensing and drug delivery techniques. In one aspect, a device for detecting an analyte and/or releasing a biochemical into a biological fluid can include an array of hollowed needles, in which each needle includes a protruded needle structure including an exterior wall forming a hollow interior and an opening at a terminal end of the protruded needle structure that exposes the hollow interior, and a probe inside the exterior wall to interact with one or more chemical or biological substances that come in contact with the probe via the opening to produce a probe sensing signal, and an array of wires that are coupled to probes of the array of hollowed needles, respectively, each wire being electrically conductive to transmit the probe sensing signal produced by a respective probe.

J. Gao, et al, Simultaneous detection of glucose, uric acid and cholesterol using flexible microneedle electrode array-based biosensor and multi-channel portable electrochemical analyzer, Sensors & Actuators: B. Chemical 287 (2019) 102-110, discloses a flexible microneedle electrode array-based biosensor (MEAB) and a multi-channel portable electrochemical analyzer (MPEA) for the simultaneous detection of glucose, uric acid, and cholesterol. Microneedle electrode array (MEA) was fabricated on the flexible substrate using magnetorheological drawing lithography, followed by sputter coated with Au/Ti film. Then, through biofunctionalized with glucose oxidase, uricase and cholesterol oxidase, MEA was conditioned as a biosensor for simultaneous detection of glucose, uric acid and cholesterol.

### SUMMARY

The present invention is defined by the claims. The techniques disclosed herein can be implemented in various embodiments to achieve devices, systems, and methods that relate to aptamer-assisted microneedle-based electrochemical sensing for label-free, continuous real-time monitoring of biomarkers in a biofluid.

One aspect of the disclosed technology relates to a device that includes a substrate. The device also includes at least two microneedles coupled to the substrate. Each microneedle in the at least two microneedles includes a protruded needle structure and an electrode probe structure. The protruded needle structure comprises an exterior wall extending outward from a surface of the substrate, the exterior wall circumscribing an interior volume of the protruded needle structure and forming an apex at a terminus point of the exterior wall. The electrode probe structure is configured to produce a signal in response to one or more chemical or biological substances in a biofluid that come in contact with the electrode probe structure. The electrode probe structure of a first microneedle in the at least two microneedles includes an aptamer sequence which is specific for a first analyte, the electrode probe structure of the first microneedle is operable as a working electrode for detection of the first analyte using a first electrochemical detection technique. The electrode probe structure of a second microneedle in the at least two microneedles is operable as an electrochemical counter electrode or an electrochemical reference electrode.

Another aspect of the disclosed technology relates to a method of manufacturing an electrochemical sensing device that includes providing a substrate. The method further includes coupling at least two microneedles to the substrate. Each microneedle in the at least two microneedles includes a protruded needle structure and an electrode probe structure. The protruded needle structure comprises an exterior wall extending outward from a surface of the substrate, the exterior wall circumscribing an interior volume of the protruded needle structure and forming an apex at a terminus point of the exterior wall. The electrode probe structure is configured to produce a signal in response to one or more chemical or biological substances in a biofluid that come in contact with the electrode probe structure. The electrode probe structure of a first microneedle in the at least two microneedles includes an aptamer sequence which is specific for a first analyte, the electrode probe structure of the first microneedle is operable as a working electrode for detection of the first analyte using a first electrochemical detection technique.

### BRIEF DESCRIPTION OF THE DRAWINGS

FIG. 1 shows a block diagram depicting an example embodiment of an electrochemical microneedle sensor device having at least two microneedle-based working electrodes.
FIG. 2 shows a block diagram depicting an example embodiment of the electrochemical microneedle sensor device of FIG. 1 in communication with an electronic device.
FIG. 3 shows a block diagram of an example embodiment of the electronic device shown in FIG. 2.
FIG. 4 shows a block diagram depicting an example embodiment of the electrochemical microneedle sensor device having at least two groups of microneedle-based working electrodes.
FIG. 5 shows a block diagram depicting an example embodiment of an electrochemical microneedle sensor device having at least three microneedle-based working electrodes.
FIG. 6 shows a block diagram depicting an example embodiment of an electrochemical microneedle sensor device having at least four microneedle-based working electrodes.
FIG. 7 shows a schematic illustration of an example embodiment of an electrochemical microneedle sensor device having at least four microneedle-based working electrodes.
FIG. 8 illustrates a diagram of an example embodiment of a method of manufacturing an electrochemical sensing device.

### DETAILED DESCRIPTION

This patent document describes design and fabrication of wearable microneedle-based devices for label-free and continuous real-time monitoring of biomarkers, including protein biomarkers, as a non-invasive diagnostic tools providing in-vivo analytical information of clinically important biomarkers in, for example, the interstitial fluid (ISF). The developed diagnostics microneedle-based device can be easily worn on the individual's skin and can measure concentrations of various biomarkers including, for example, protein biomarkers in a real-time mode directly inside the ISF.

The analysis is performed on the aptamer-modified electrodes which are in the form of microneedles (e.g., polymeric hollow microneedles) and acting as electrochemical sensors. Aptamers functionalized by a redox reporter molecule (e.g. methylene blue or anthraquinone) specifically and reversibly bind to the analyte of interest, upon which a folding in the conformation of the electrode-bound aptamer occurs. This binding-induced folding leads to a change in the electron transfer characteristics of the redox molecule that is corresponding to the target biomarker's concentration and is detected using electrochemical techniques, e.g. square wave voltammetry (SWV).

While the present document describes application of the disclosed technology to real-time aptamer-assisted measurement of cortisol and insulin biomarkers as model analytes, the technology disclosed in this patent document is readily expandable to the continuous monitoring of any biomarkers such as, for example, hormone, and proteins of interest through binding to their relevant aptamer-based biorecognition elements.

The in-vivo stable electrochemical detection of the target analytes present in the ISF can be achieved, according to the technology disclosed herein, with no surface fouling through using an effective anti-biofouling self-assembled monolayer on a surface of a microneedle electrode or a hydrogel coating. Furthermore, the aptamers can be rationally engineered in-vitro to implement the analyte detection in a desired concentration window.

Unlike microneedle-based enzymatic sensing, the disclosed technology expands the capabilities of microneedle sensors to widen the scope of biomarkers that can be detected. Sensing of such biomarkers commonly rely on immunoassays which are not always feasible to perform at the microneedle tip.

Another highly important aspect of the disclosed technology is the integration of aptamer-based electrochemical sensing with enzymatic sensing on a single microneedle array device toward continuous real-time reversible monitoring of multiple biomarkers simultaneously.

Currently there is no available device for the minimally-invasive or non-invasive continuous monitoring of various biomarkers and the only methods available for measuring biomarkers are based on the traditional ways of sampling blood using hypodermic needles followed by quantification using external meters/techniques in centralized laboratories (mostly using ELISA method which is highly time-consuming and expensive). Such methods have significant disadvantages, including patient discomfort, the need for relatively high sample volume, and the generation of sharp waste. More importantly, they cannot track trends and fluctuations in the levels of biomarkers and thus, cannot provide real-time information regarding the biomarker concentrations.

There is currently no viable technology to provide real-time on-body information regarding concentrations of different biomarkers and developing a wearable multiplexed sensor capable of continuously monitoring the dynamically changing levels of different disease markers at the same time would be of critical significance for disease diagnosis, prognosis, and treatment.

There have been many examples of sensors that detect physiologically relevant biomarkers in-vitro, but yet there is no example of biosensors for in-vivo detection of ISF biomarkers. Wearable on-body biosensing has a potential to revolutionize healthcare by enabling personalized medicine. Through providing clinically relevant health information on a continuous basis using wearable non-invasive sensors, an individual's health conditions can be well understood, providing a platform to disease diagnosis, prognosis and management. The present document shows examples of wearable on-body sensor devices for continuous real-time monitoring of various biomarkers which obviate the cost and complexity of traditional immunoassay-based approaches. An example microneedle device based on the technology disclosed in this patent document can be used for multiplexed monitoring of different biomarkers such as, for example, cortisol, insulin, ketone bodies, and glucose for diabetic patients. Such a device can be in a form of a fully-integrated wearable microneedle patch which can be used for self-monitoring and/or used by healthcare providers. The aptamer-based microneedle sensing protocol described herein can be readily expanded to the detection of other biomarkers of interest. This takes a leap forward in the field of disease diagnostics and personalized healthcare.

The microneedle aptamer-based electrochemical sensing devices described herein are the first demonstration of combining electrochemical aptamer-based sensing with microneedle devices for diagnostic applications. This novel electrochemical platform is also the first example of a wearable on-body patch capable of non-invasively monitoring different biomarkers in the ISF.

Various embodiments of the microneedle based sensor devices according to the technology disclosed in this patent document integrate sensing of biomarkers using aptamers with other sensing modalities and/or strategies, including, but not limited to, enzymatic detection of important metabolite biomarkers on a single microneedle array platform, as it has been shown herein on the example of simultaneous detection of four important diabetes-related analytes, i.e. cortisol, insulin, ketone bodies, and glucose.

The application of microneedles for diagnostic and monitoring purposes has received much attention over the last decade in view of the rich molecular information contained in the interstitial fluid. However, direct sensing of the ISF markers at a microneedle tip is usually carried out using enzyme-modified microneedle electrode transducers. These rely on reversible biocatalytic reactions of the corresponding substrates.

In contrast, implementation of common bioaffinity assays, such as immunoassays, at the microneedle tips faces a barrier owing to the strong interaction between the target antigen and the capture antibody which makes the regeneration of the antibody receptor extremely difficult, along with the need for several washing and incubation steps. Often such assays also require additional incubation steps with related tagged reagents to perform competitive or sandwich assays, which are not always feasible to perform at the tips of microneedles.

The only reports on the microneedle-assisted antibody-based biomarkers analysis are either based on the sampling the ISF including the biomarkers or the selective capture of specific biomarkers through their binding to the antibodies functionalized on the surface of microneedles, followed by off-body analysis procedures. None of these reports have the capability to adapt to the continuous monitoring of the biomarkers directly at the microneedle tip. As a result, the use of microneedle ISF sensors have not been reported for in-vivo bioaffinity monitoring of ISF biomarkers and the realization of in-situ real-time bioaffinity assays on the microneedle tip toward ISF monitoring of various markers remains as a major challenge.

The present document describes the realization of real-time bioaffinity assays on a microneedle tip electrode through the use of reversible binding, conformation-dependent aptamers.

Aptamers have recently shown great promise for the real-time and continuous monitoring of therapeutic drugs in live animals using electrochemical approaches. However, these aptamers have been reported toward continuous monitoring of drugs and other small analytes only, and they have not been used for in-vivo monitoring of higher-molecular-weight analytes (e.g., proteins etc.). Also, they have been applied through invasive methods using, for example, 18-gauge catheters.

By combining the microneedles and aptamer-based electrochemical sensing in the disclosed technology, we demonstrate the first example of in-situ bioaffinity-based monitoring of biomarkers (including protein biomarkers) in a non-invasive fashion. The disclosed technology can be used for highly sensitive and stable real-time monitoring of any biomarker of interest in connection with the corresponding aptamer biorecognition element.

Another unique feature of the technology disclosed in this patent document is the simultaneous multiplexed detection of biomarkers (e.g., metabolites) using non-aptamer substrates (e.g., enzymatic substrates) within a single microneedle array platform. The combination of different surface chemistries and sensing modalities toward on-body continuous monitoring of various markers has been a long-sought goal. The smart architecture of different surface chemistries employed in the devices according to the technology disclosed herein enables a user-friendly approach toward the multiplexed wearable combination of these various sensing formats. The devices and methods reported herein for multiplexed detection of cortisol, insulin, glucose and ketone bodies can be applied/expanded to cover other biomarkers (e.g., proteins//metabolites/drugs/electrolytes) of interest. Also, the technology disclosed in this patent document allows designing a closed-loop system capable of continuously monitoring concentrations of various biomarkers (disease-related or otherwise) and regulating delivery of therapeutic agents based on the measured biomarker concentrations.

These and other example features of the devices, systems, and methods in accordance with the technology disclosed in this patent document are described below.

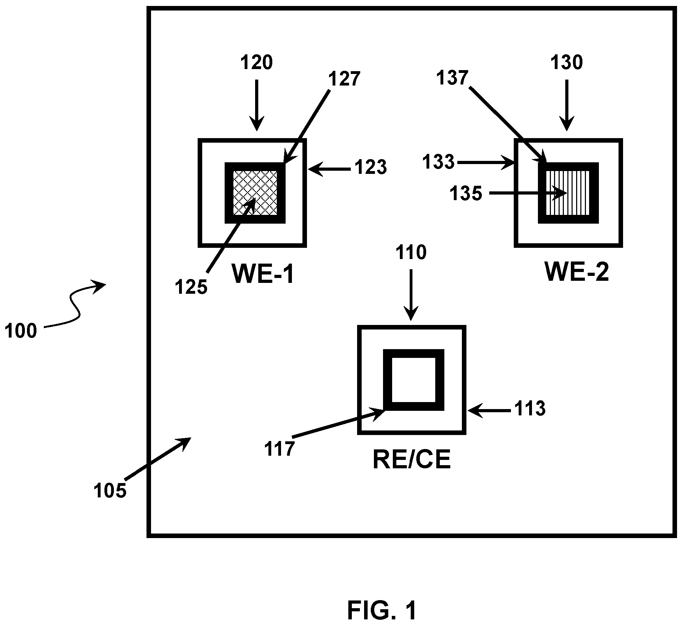

FIG. 1 shows a block diagram depicting an example embodiment of an electrochemical microneedle sensor device 100 having at least two microneedle-based working electrodes for sensing of two different analytes in accordance with the present technology. In the device 100, the at least two microneedle-based working electrodes are disposed on a substrate proximate to a reference and/or counter microneedle-based electrode. As shown in the block diagram in FIG. 1, the device 100 can include a first microneedle electrode 110 configured as a reference electrode, RE, and/or counter electrode, CE, for electrochemical sensing of both the first analyte and the second analyte. The electrode 110 is disposed on a substrate 105. The device 100 can include a second microneedle electrode 120 configured as a first working electrode, WE-1, and a third microneedle electrode 130 configured as a second working electrode, WE-2. The electrodes 120 and 130 are disposed on the substrate 105 as well. The microneedle electrodes 110, 120, and 130 of the electrochemical microneedle sensor device 100 include a microneedle structure and an electrode probe structure. The diagram of FIG. 1 shows microneedle structures 113, 123, and 133 of the microneedle electrodes 110, 120, and 130, respectively. The microneedle structure can include, for example, an exterior wall spanning outward from a base surface (for example, the base surface can be located at the surface of the substrate 105) and forming an apex at a terminus point of the exterior wall. For example, at a portion of the exterior wall, there can be an opening leading into a hollow interior (or cavity) of the needle structure defined by an interior wall. In some embodiments, the microneedle structure can have a pyramidal geometry (e.g., trigonal pyramidal with three exterior walls or square pyramidal with four exterior walls). In other embodiments, the microneedle structure can have a conical geometry. In yet other example embodiments, at least two microneedle structures of the device 100 can have different geometries. For example, in an embodiment of the device 100, the microneedle structure of the electrode 110 can have a conical geometry, the microneedle structure of the electrode 120 can have a trigonal pyramidal geometry, and the microneedle structure of the electrode 130 can have a square pyramidal geometry.

In various example embodiments, the electrode probe structure is disposed (completely or partially), for example, within the interior or cavity of the respective microneedle structure. In other example embodiments, the electrode probe structure is incorporated with or attached to the respective microneedle structure and positioned (completely or partially) outside the interior or cavity of the respective microneedle structure. In yet other example embodiments, the electrode probe structure is incorporated with or attached to the respective microneedle structure, wherein the respective microneedle structure does not have the mentioned interior or cavity. The diagram of FIG. 1 shows electrode probe structures 117, 127, and 137 of the microneedle electrodes 110, 120, and 130, respectively. For example, the electrode probe structure of an electrode of the microneedle sensor device 100 can include a silver (Ag), gold (Au) or platinum (Pt) wire or film and/or carbon paste (CP).

The substrate 105 used in example embodiments of the electrochemical microneedle sensor device 100 can include an electrically insulative material, such as, for example, a plastic material (e.g., polyethylene terephthalate (PET), polyethylene terephthalate glycol (PETG), polyethylene naphthalate (PEN), polyimide (PI), or other). The substrate 105 can be flexible and/or bendable and/or stretchable; and/or the substrate 105 can include an adhesive on at least one side of the substrate 105 to enable attachment of the device 100 to a subject, e.g., via attachment to the subject's to skin.

In some implementations, the height of the microneedle structures (e.g., base to apex) can be in a range of 1 mm to 2 mm, e.g., preferably about 1.5 mm. In some implementations, the diameter or width of the microneedle structure can be between 200 µm and 500 µm. The microneedle structures can be spaced at various spacings and arrangements, which can be selected based on the application of the microneedle sensor device 100. In some implementations, the microneedle structures are spaced apart in the tens of microns or hundreds of microns, base-to-base; for example, in some implementations, the microneedle structures are spaced about 1 mm apart from apex-to-apex. In some implementations, the microneedle structures are arranged on the substrate 105 in a linear array; whereas in some implementations the microneedle structures are arranged on the substrate 105 in a circular array, a rectangular array, a triangular array, or any other patterned or non-patterned arrangement.

The microneedle structures and electrodes can be configured in other arrangements, e.g., as described in U.S. Patent No. 9,737,247 which is incorporated herein by reference in its entirety as part of the disclosure of this patent document.

In some example embodiments, depending on the electrochemical sensing technique to apply at the particular working electrode, the second microneedle electrode 120 and the third microneedle electrode 130 can include one or more functionalization materials. In FIG. 1, the second microneedle electrode 120 includes a functionalization material 125 disposed on or integrated with at least a portion of the electrode probe structure 127. In some embodiments, the electrode probe structure 127 functionalized with the functionalization material 125 can be located (e.g., at least partially) within the opening or cavity of the microneedle structure 123. Similarly, in some embodiments, the third microneedle electrode 130 includes a functionalization material 135 disposed on or integrated with at least a portion of the electrode probe structure 137. In some embodiments, the electrode probe structure 137 functionalized with the functionalization material 135 can be located within the opening or cavity of the microneedle structure 133.

For example, in some embodiments, materials of the same type or of different types can be used to manufacture electrode probe structures 117, 127, and 137 of the microneedle electrodes 110, 120, and 130, respectively, e.g. for varying detection purposes. For example, the electrode probe structure 117 can include a metal Ag/AgCl wire (e.g., about 500 microns in diameter) incorporated within the interior of the microneedle structure 113 of the microneedle electrode 110 and can acts as a RE. For example, the electrode probe structure 127 can include a gold wire (e.g., about 500 microns diameter) incorporated within the cavity of the microneedle structure 123 of the microneedle electrode 120 and be employed as a WE for detection of a protein biomarker, for example. The gold wire of the electrode probe structure 127 can be insulated with parylene (a poly(para-xylylene) polymer) conformal coating, for example. The parylene coating can create a highly uniform, pinhole-free and chemically-resistant coating of, e.g., about 30 microns thick for the Au wire. The parylene coating can serve multiple purposes including electrical insulation, chemical isolation, and mechanical protection of the wire, for example. The parylene coating of the gold wire of the electrode probe structure 127 can be configured to expose a reproducible surface area of the wire for conduction of electrochemical measurements.

High-affinity aptamer sequences, developed using, for example, systematic evolution of ligands by exponential enrichment (SELEX) approach to be specific for a target analyte (e.g., to be able to bind to the target analyte (e.g., a biomarker such as insulin) in, for example, a reversible fashion), can be tethered to the Au wire of the electrode probe structure 127 via 5'-thiol links, for example. The aptamer sequences are functionalized with a redox reporter molecule, e.g. methylene blue or anthraquinone, at the 3' end or 5' end of the aptamer sequence. The functionalization of the aptamer sequences may be such that selective binding of the target biomarker to the aptamer sequence would bring the redox reporter closer to the surface of the Au wire of the electrode probe structure 127 to facilitate electron transfer between the reporter molecule and the electrode probe structure 127.

Aptamers functionalized by a redox reporter molecule (e.g., methylene blue or anthraquinone) can specifically and reversibly bind to a compound of interest, upon which a folding in the conformation of the electrode-bound aptamer occurs. This binding-induced folding can lead to a change in the electron transfer characteristics of the redox molecule that is corresponding to the concentration of the target compound of interest and is detected using electrochemical detection techniques such as square wave voltammetry (SWV) implemented for aptamer-functionalized electrodes of the sensor device 100. The multiplexed detection of different biomarkers can be achieved through labeling aptamers with different redox reporters having different redox characteristics or by spatially localized measurement of current signals of a single redox tag.

Electrochemical detection modalities or measurement techniques other than square wave voltammetry can be employed for detection of a target analyte at any of the working microneedle electrodes 120 or 130 of the sensor device 100. Among the possible electrochemical measurement techniques that can be employed by the sensor device 100 the following ones can be mentioned.

A potentiometric measurement technique is the one where an open circuit potential of the electrochemical cell is measured directly. This potential can be measured between a reference electrode (e.g., the reference electrode 110 of the sensor device 100) and a working electrode (e.g., the working electrode 120 or the working electrode 130 of the sensor device 100).

The potentiometric measurement technique is contrasted with the amperometric family of measurements that measure current while controlling the cell potential. For example, chronoamperometry is a powerful tool for measuring diffusion-controlled reactions. In chronoamperometry, the potential is stepped at the beginning of a measurement and then remains constant throughout the duration of the measurement. The current that results from this stimulus is plotted as a function of time.

Voltammetry techniques vary the potential as a function of time. The resulting current is plotted as a function of potential. For example, cyclic voltammetry (CV) sweeps the potential of the cell linearly across a voltage range, while a fast scan CV (FSCV) technique does this at a faster rate. Square-wave voltammetry (SWV) uses a square wave superimposed over a staircase function to provide a sweeping measurement that provides two sampling instances per potential. As a result of this sampling technique, the contribution to the total current that results from non-faradic currents is minimized. Like CV, the current is plotted as a function of potential.

In some embodiments, the electrode probe structure 137 of the microneedle electrode 130 can be structured in the same or in a similar way to the electrode probe structure 127 of the microneedle electrode 120. The aptamer sequences tethered to the Au wire of the electrode probe structure 137 can be tailored to bind to an analyte different from the target analyte which is specific to the aptamer sequences that are tethered to the Au wire of the electrode probe structure 127. For example, the aptamer sequences tethered to the Au wire of the electrode probe structure 137 can be configured to bind to cortisol. Note that, similar to insulin, cortisol is an important diabetes-related biomarker. As such, the microneedle electrode 120 of the device 100 can be configured to detect a first analyte (e.g., insulin), while the microneedle electrode 130 of the device 100 can be configured to detect a second analyte (e.g., cortisol) that is different from the first analyte.

In certain embodiments of the device 100, the aptamer sequences of the electrode probe structure 127 can be functionalized with a first type of redox reporter molecule (e.g. methylene blue) while the aptamer sequences of the electrode probe structure 137 can be functionalized with a second type of redox reporter molecule (e.g., anthraquinone) that is different from the first type of redox reporter molecule. In other embodiments, both the aptamer sequences of the electrode probe structure 127 and the aptamer sequences of the electrode probe structure 137 can be functionalized with the same type of redox reporter molecule (e.g., one of methylene blue or anthraquinone). Different types of reporter molecules used to functionalize the aptamer sequences can provide different redox characteristics that can be used to perform multiplexed (simultaneous or otherwise) measurements of different target compounds.

Potentially high biofouling of the device 100 when it is working in complex media, such as ISF can be avoided through applying an (outer) polymer membrane coating (e.g., polysulfone or zwitterionic polymer coating) to the electrode probe structures of the microneedle electrodes of the microneedle sensor device 100.

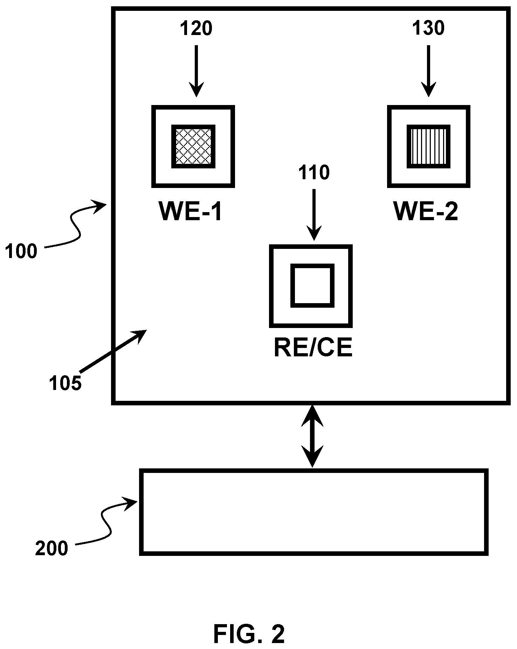

FIG. 2 shows a block diagram depicting an example embodiment of the electrochemical microneedle sensor device 100 of FIG. 1 in communication with an electronic device 200, in accordance with the present technology.

It should be noted that while, for simplicity of explanation, the same reference numbers have been used to identify some of the elements in different figures (e.g., electrodes 110, 120, and 130 in FIGS. 1 and 2), it is understood that these designations do not necessarily mean that those elements are identical. In particular, one or more characteristics of the elements (e.g., physical, chemical, electrochemical, material, dimension, etc.) are contemplated to be modified based on the particular configuration of the microneedle sensor device according to the technology disclosed in this patent document and/or based on its desired performance characteristics.

In various embodiments, for example, the electronic device 200 can affect control, measurement, and monitoring functions, among other functions, of the sensor device 100 that are related to operation of the sensor device 100. In some example implementations, the electronic device 200 can be included in an electronics unit of a wearable medical device that incorporates the sensor device 100 or is otherwise interfaced with the sensor device 100. For example, in some embodiments, the electronics unit 200 is configured on the same substrate 105 as the sensor device 100, allowing for an all-in-one sensor patch to be implemented for accurately detecting concentrations of target analytes (e.g., insulin and cortisol) using multiplexed (simultaneous (parallel) or sequential) detection of the target analytes using different electrodes or different groups of electrodes for detection of the respective analytes.

For example, in some embodiments, the electrochemical microneedle sensor device 100 is configured on a patch, which is attachable and conformable on a user's skin for mobile, remote monitoring applications. The patch can integrate the sensor device 100 as well as the electronic device 200 on a single substrate (e.g., the substrate 105) or a single transferrable platform.

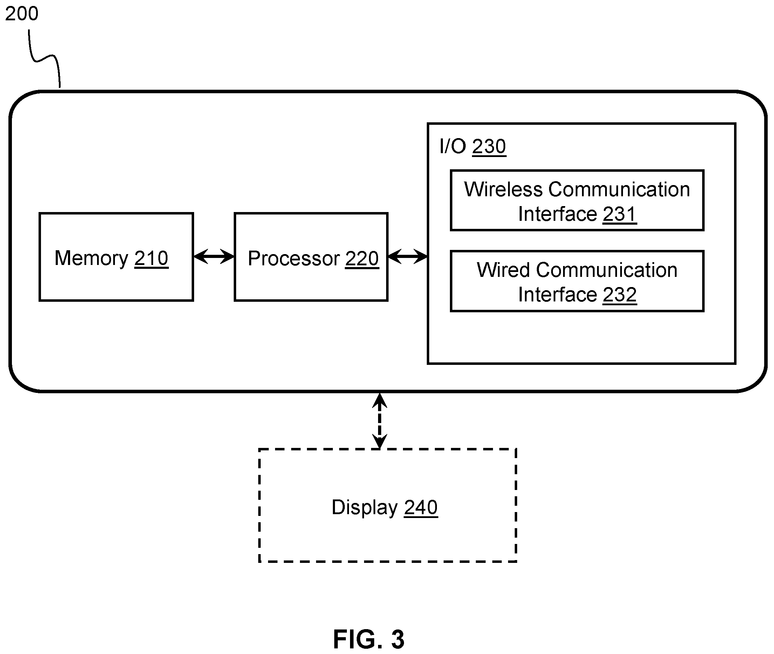

FIG. 3 shows a block diagram of an example embodiment of the electronic device 200. For example, the electronic device 200 can be configured to be electrically coupled to at least one electrode (or electrode probe structure) of the sensor device 100. For example, the electronic device 200 can be configured to supply signals (e.g., voltage and/or current) across at least one electrode (or electrode probe structure) of the device 100. The electronic device 200 can be also configured, for example, to receive and process electric signals (e.g., voltage, current) produced by the at least one electrode (or electrode probe structure) of the sensor device 100. The electronic device 200 can be further configured, based on the signals obtained by the device 200 from one or more electrodes (or electrode probe structures) of the device 100, to determine concentration of one or more target analytes present in the ISF and/or other fluids and/or tissues of a body that are in contact with at least one electrode (or electrode probe structure) of the sensor device 100.

In some embodiments, the electronic device 200 can address electrodes (e.g., apply signals across these electrodes or acquire signals from these electrodes or both) of the device 100 configured to detect a first target analyte (e.g., electrode 120 configured to detect insulin) independently from the electrodes of the device 100 configured to detect a second target analyte that is different from the first target analyte (e.g., electrode 130 configured to detect cortisol). For example, the electronic device 200 can provide electric connections to the electrodes of the device 100 configured to detect the first target analyte such that these connections are independent from the electric connections provided by the electronic device 200 to the electrodes of the device 100 configured to detect the second target analyte. For example, the electronic device 200 can address the electrodes of the device 100 that are configured to detect (that correspond to) different analytes at the same time (simultaneously) but in such a manner that electric signals applied to and/or acquired from the electrodes that correspond to different analytes do not mix with each other. Alternatively, or in addition to the manner of addressing electrodes of the device 100 just mentioned, the electronic device 200 can acquire electric signals from the electrodes of the device 100 that correspond to different analytes in a manner which provides a mixture of signals corresponding to different analytes and then perform processing of the mixture of signals to separate it into components corresponding to the respective individual analytes. In some embodiments, the electronic device 200 can address the electrodes of the device 100 that correspond to different analytes in a sequential manner when the electrodes corresponding to the first target analyte are addressed first (e.g., signals applied across these electrodes or signals acquired from these electrodes or both) followed by addressing the electrodes that correspond to the second target analyte (e.g., signals applied across these electrodes or signals acquired from these electrodes or both).

In some embodiments, the electronic device 200 can apply signals across electrodes of the device 100 that correspond to different analytes using a single signal source of the electronic device 200. For example, the single signal source of the electronic device 200 can be used to apply signals (e.g., directly or through one or more passive or active electric elements such as, for example, resistors, capacitors, transistors, diodes, etc.) across one or more electrodes of the device 100 that correspond to a first target analyte (e.g., insulin) and the single signal source can be also used to apply the same or different signals across one or more electrodes of the device 100 that correspond to a second target analyte (e.g., cortisol). In some implementations, the single signal source of the electronic device 200 can generate a signal that is simultaneously provided to (e.g., directly or through one or more passive or active electric elements) one or more electrodes of the device 100, including electrodes that correspond to different target analytes. As another example, the single signal source of the electronic device 200 can first generate a signal corresponding to the electrodes configured to detect the first target analyte and then generate a signal corresponding to the electrodes configured to detect the second target analyte. In other embodiments, the electronic device 200 can include multiple signal sources and can apply signals in a parallel manner (e.g., substantially simultaneously) or in a sequential manner across the electrodes of the device 100 corresponding to different analytes using different signal sources.

Similarly, the electronic device 200 can include a single data acquisition element (e.g., an analog-to-digital converter (ADC)) or multiple data acquisition elements that can be used to acquire electric signals from the electrodes of the device 100. In some embodiments, the electronic device 200 can include a first data acquisition element configured to acquire electric signals from the electrodes of the device 100 that correspond to a first target analyte and the electronic device 200 can include a second data acquisition element configured to acquire electric signals from the electrodes of the device 100 that correspond to a second target analyte. In some implementations, the electronic device 200 can perform signal acquisition from the electrodes of the device 100 that correspond to the first target analyte using the first data acquisition element simultaneously (or, in some implementations, substantially simultaneously) with performing signal acquisition from the electrodes of the device 100 that correspond to the second target analyte using the second data acquisition element. In other implementations, the electronic device 200 can perform signal acquisition from the electrodes of the device 100 that correspond to the first target analyte using a data acquisition element followed by performing signal acquisition from the electrodes of the device 100 that correspond to the second target analyte using the same data acquisition element. In yet other implementations, the electronic device 200 can perform signal acquisition from the electrodes of the device 100 that correspond to the first target analyte using a data acquisition element simultaneously with performing signal acquisition from the electrodes of the device 100 that correspond to the second target analyte using the same data acquisition element.

Although the examples above include electrodes of the device 100 configured to detect two different target analytes, the technology disclosed in this patent document can be applied to detection of any number of the target analytes. Accordingly, the device 100 can have any number of electrodes configured to detect any number of target analytes.

In various implementations, the electronic device 200 is operable to store and execute software applications and algorithms to process signals obtained by the electronic device 200 from the sensor device 100 and implement various controls of the sensor device 100, such as application of various voltage waveforms across one or more electrodes of the sensor device 100, for example. In various embodiments, the electronic device 200 can be implemented as a portable computing device, such as a mobile communications device, such as a smartphone, tablet or wearable device, like a smartwatch, glasses, etc.; and/or the electronic device 200 can be implemented as a stationary computing device, such as a desktop computer.

In some embodiments, the electronic device 200 includes a processor 220 configured to process data, a memory 210 in communication with the processor 220 configured to store data, and an input/output (I/O) communication interface (or unit) 230 configured to interface the processor 220 and/or the memory 210 to other elements of the electronic device 200 as well as to various modules, units, or devices, including the device 100 and/or external computing devices, data storage devices, or communication devices, for example.

In some embodiments, the I/O unit 230 is electrically interfaced with at least one electrode of the device 100. In some implementations, the I/O 230 includes an analog-to-digital (ADC) converter that converts an electric signal (e.g., current or voltage) received by the I/O 230 from an electrode of the device 100 into a digital form suitable for processing by the processor 220 and/or storage inside the memory 210. In some implementations, the I/O 230 includes a digital-to-analog (DAC) converter that, for example, converts a digital waveform obtained by the processor 220 from the memory 210 into a voltage waveform that is applied across an electrode of the device 100 to implement, using the electrode, one of the electrochemical measurement techniques described above or any other electrochemical measurement or detection technique. In some embodiments, the I/O unit 230 can interface the processor 220 and/or the memory 210 to other modules, units or devices, including other external computing devices.

For example, the processor 220 can include a central processing unit (CPU) or a microcontroller unit (MCU) or a graphics processing unit (GPU). For example, the memory 210 can include and store processor-executable code, which, when executed by the processor 220, configures the electronic device 200 to perform various operations, e.g., such as receiving information, commands, and/or data, processing information, commands, and/or data, and transmitting or providing information, commands, and/or data to another element of the electronic device 200 and/or to another device external to the electronic device 200.

In some implementations, the electronic device 200 can transmit raw or processed data (e.g., voltage profiles captured by the electronic device 200 from one or more electrodes of the device 100) to a computer system or a computer network which can include one or more remote computational processing devices (e.g., servers) and which can be accessible via a communication network such as the Internet (such computer systems or networks are sometimes referred to as being located "in the cloud").

To support various functions of the electronic device 200, the memory 210 can store information and data, such as instructions, software, values, voltage or current profiles, and other data processed or referenced by the processor 220. For example, various types of Random Access Memory (RAM) devices, Read Only Memory (ROM) devices, Flash Memory devices, and other suitable storage media can be used to implement storage functions of the memory 210.

In some embodiments, the I/O unit 230 includes a wireless communication interface 231, such as a wireless transmitter configured to transmit stored and/or processed data, for example, or a wireless transceiver (Tx/Rx) configured to transmit and receive data. For example, in some embodiments, the I/O unit 230 can also include a wired communication interface 232, which can be used to exchange electric signals between elements of the electronic device 200 as well as between the electronic device 200 and the device 100, for example. The I/O unit 230 can utilize various types of wired or wireless interfaces compatible with typical data communication standards, for example, which can be used in communications of the electronic device 200 including, but not limited to, Bluetooth, Bluetooth low energy, Zigbee, IEEE 802.11, Wireless Local Area Network (WLAN), Wireless Personal Area Network (WPAN), Wireless Wide Area Network (WWAN), WiMAX, IEEE 802.16 (Worldwide Interoperability for Microwave Access (WiMAX)), 3G/4G/LTE/5G cellular communication methods, NFC (Near Field Communication), and parallel interfaces. In some embodiments, the electronic device 200 uses its I/O unit 230 for the purpose of data transfer to another device as well as for receiving data (e.g., voltage profiles to be applied across one or more electrodes of the sensor device 100 during its operation) from another device.

In some embodiments, the electronic device 200 includes or is otherwise interfaced with a display unit 240, which can include a visual display such as a display screen, an audio display such as a speaker, or any other type of display or combinations thereof.

The I/O unit 230 of the electronic device 200 can also interface with other external interfaces, sources of data, data storage devices, and/or visual or audio display devices, etc. to retrieve and transfer data and information that can be processed by the processor 220, stored in the memory 210, or exhibited on an output unit (e.g., the display unit 240) of the electronic device 200 or an external device. For example, the display unit 240 can be configured to be in data communication with the electronic device 200, e.g., via the I/O unit 230, to provide a visual display, an audio display, and/or other sensory display that produces the user interface of a software application. In some examples, the display unit 240 can include various types of screen displays, speakers, or printing interfaces, e.g., including but not limited to, light emitting diode (LED), or liquid crystal display (LCD) monitor or screen, cathode ray tube (CRT) as a visual display; audio signal transducer apparatuses as an audio display; and/or toner, liquid inkjet, solid ink, dye sublimation, inkless (e.g., such as thermal or UV) printing apparatuses, etc.

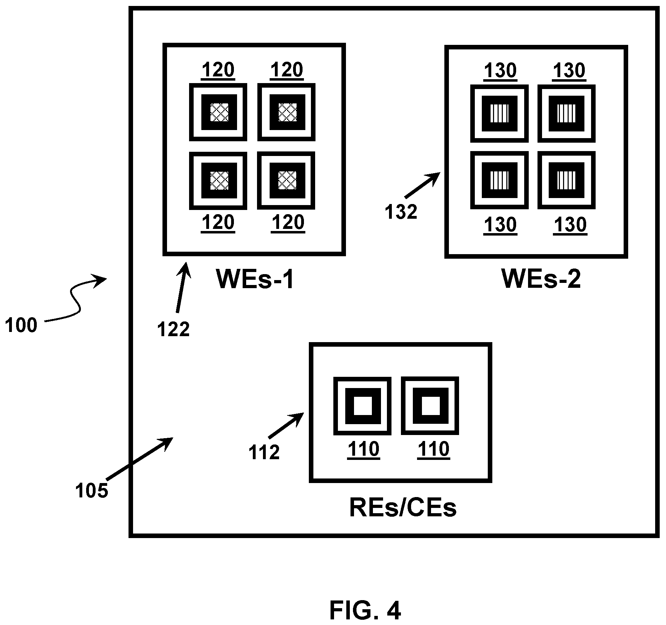

FIG. 4 shows a block diagram depicting an example embodiment of the electrochemical microneedle sensor device 100 having at least two groups of microneedle-based working electrodes for sensing of two different analytes in accordance with the technology disclosed herein. As shown in FIG. 4, the electrochemical microneedle sensor device 100 can have a group 122 of the working electrodes 120 ("WEs-1" in FIG. 4), wherein each electrode in the group 122 is configured to measure concentration of a first target analyte (e.g., insulin) in the ISF, for example, using an aptamer-assisted electrochemical detection method. As also demonstrated in FIG. 4, the electrochemical microneedle sensor device 100 can have a group 132 of the working electrodes 130 ("WEs-2" in FIG. 4), wherein each electrode in the group 132 is configured to measure concentration of a second target analyte (e.g., cortisol) that is different from the first target analyte in the ISF, for example, using an aptamer-assisted electrochemical detection method. As further demonstrated in FIG. 4, the electrochemical microneedle sensor device 100 can have a group 112 of the electrodes 110, wherein each electrode in the group 112 can be configured to be used as a reference electrode (RE) or as a counter electrode (CE) in cooperation with any electrode or multiple electrodes in any of the groups of electrodes 122 or 132. The electrode groups 112, 122, or 132 can have any numbers of corresponding electrodes which can be different from the numbers of electrodes in these groups shown in FIG. 4.

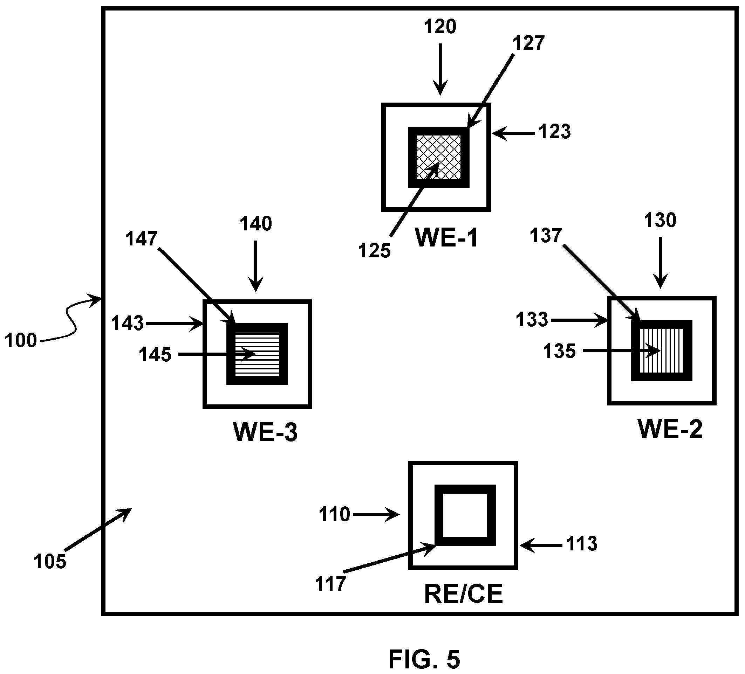

FIG. 5 shows a block diagram depicting another example embodiment of the electrochemical microneedle sensor device 100 having at least three microneedle-based working electrodes for multiplexed sensing of different target analytes in accordance with the present technology, in which the three microneedle-based working electrodes are disposed on a substrate proximate to a reference and/or counter microneedle-based electrode. As shown in the block diagram in FIG. 5, the device 100 can include a first microneedle electrode 110 configured as a reference electrode, RE, and/or counter electrode, CE, for electrochemical sensing of one or more analytes, wherein the electrode 110 is disposed on a substrate 105. The device 100 can include a second microneedle electrode 120 configured as a first working electrode, WE-1, a third microneedle electrode 130 configured as a second working electrode, WE-2, and a fourth microneedle electrode 140 configured as a third working electrode, WE-3, each disposed on the substrate 105. Each of the microneedle electrodes 110, 120, 130, and 140 of the electrochemical microneedle sensor device 100 includes the microneedle structure and the electrode probe structure, as previously described. The diagram of FIG. 5 shows microneedle structures 113, 123, 133 and 143 of the microneedle electrodes 110, 120, 130, and 140, respectively. In various example embodiments, the electrode probe structure of any of the electrodes 110, 120, 130, or 140 can be disposed (e.g., at least partially) within the interior or within the opening or cavity of the microneedle structure of the respective electrode. In other embodiments, the electrode probe structure of any of the electrodes 110, 120, 130, or 140 can be attached to or otherwise incorporated with the microneedle structure of the corresponding electrode that does not have the opening or cavity. The diagram in FIG. 5 shows electrode probe structures 117, 127, 137, and 147 of the microneedle electrodes 110, 120, 130, and 140, respectively.

In some embodiments, for example, depending on the electrochemical sensing technique to apply at the particular working electrode, the second microneedle electrode 120, the third microneedle electrode 130, and the fourth microneedle electrode 140 can include one or more functionalization materials. In FIG. 5, the second microneedle electrode 120 includes a functionalization material 125 disposed on or integrated with at least a portion of the electrode probe structure 127. Similarly, in some embodiments, the third microneedle electrode 130 includes a functionalization material 135 disposed on or integrated with at least a portion of the electrode probe structure 137. Also, in some embodiments, the fourth microneedle electrode 140 includes a functionalization material 145 disposed on or integrated with at least a portion of the electrode probe structure.

For example, in some implementations, the functionalization material 125 can include an aptamer designed to have a conformation that can receive an analyte in a biofluid, e.g., insulin in the ISF, such that an aptamer-analyte binding complex causes a conformational change of the functionalization material 125 that is detected at the electrode 120 as an electrical signal. The functionalization material 125 can include, for example, a redox reporter molecule, e.g. methylene blue or anthraquinone. Processing of the detected electrical signal can provide information about concentration or level of the analyte (e.g., insulin) in the biofluid (e.g., the ISF). Similarly, in some implementations, the functionalization material 135 can include an aptamer designed to have a conformation that can receive another analyte in the biofluid, e.g., cortisol in the ISF, such that an aptamer-analyte binding complex causes a conformational change of the functionalization material 135 that is detected at the electrode 130 as an electrical signal. The functionalization material 135 can include, for example, the same type of redox reporter molecule that is included in the functionalization material 125 (e.g. methylene blue or anthraquinone), or the functionalization material 135 can include a different type of redox reporter molecule.

For example, in some implementations, the electrode probe structure 147 of the microneedle electrode 140 can be functionalized with a biocatalyst 145 such as an enzyme, e.g., glucose oxidase (GOx), or other, that, for example, can be attached to the electrode 140 via electropolymeric entrapment. The enzyme (e.g., GOx) can be configured to be specific to a target analyte (e.g., glucose) present in a biofluid (e.g., the ISF or blood) or a body tissue. In such embodiments, for example, enzyme-based detection of the target analyte, (e.g., glucose) can be performed using the functionalized microneedle electrode 140. In other implementations, non-enzymatic detection of a target analyte can be performed using, for example, the electrode 140 in which the electrode probe structure 147 includes a metal (e.g., gold) wire at least partially covered by a graphene film functionalization material 145. In yet other embodiments, the functionalization material 145 of the electrode 140 can include β-hydroxybutyrate dehydrogenase (HBD) enzyme to enable detection of ketone bodies at the electrode 140. As another example, the functionalization material 145 can include carbon (e.g., graphite) powder along with a binder (such as an oil) and an enzyme which is specific to a target analyte (e.g., glucose or ketone bodies). As yet another example, the functionalization material 145 can include a carbon paste-based functionalization material having catalytic metal nanoparticles embedded in it to enhance a detection signal using a non-enzymatic type of detection of a target analyte. The functionalization materials 125, 135, and 145 of the microneedle sensor device 100 depicted in FIG. 5 can include any of the functionalization materials described earlier or any other functionalization materials suitable for detection of the target analytes at any of the functionalized electrodes 120, 130, and/or 140 using any of the electrochemical measurement techniques described above.

Note that the target analytes mentioned above can share a common property of being biomarkers related to a certain disease such as diabetes, for example, or being indicative of the condition of one or more physiologic systems and/or organs of a body.

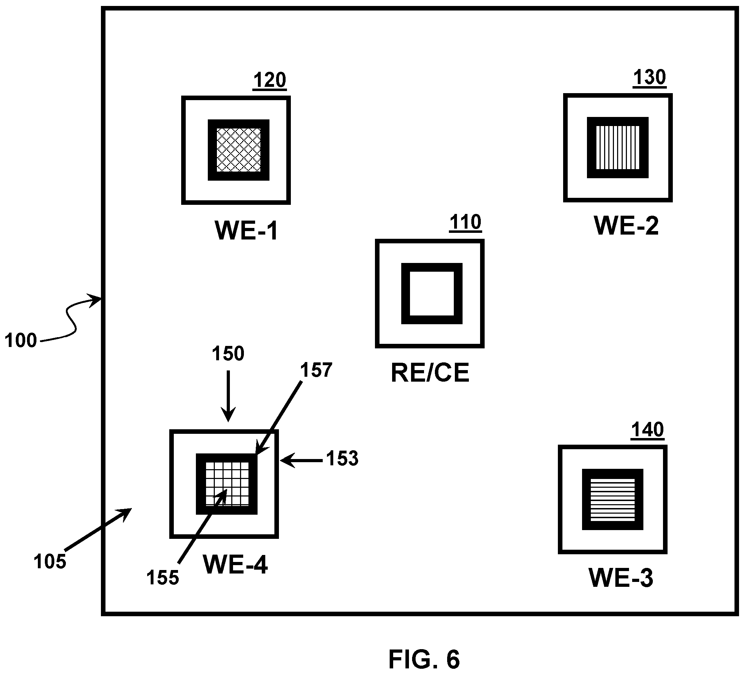

FIG. 6 shows a block diagram depicting another example embodiment of the electrochemical microneedle sensor device 100 having at least four microneedle-based working electrodes for multiplexed sensing of different target analytes in accordance with the technology disclosed in this patent document, in which the four microneedle-based working electrodes are disposed on a substrate proximate to a reference and/or counter microneedle-based electrode. As shown in the diagram in FIG. 6, the device 100 can include a first microneedle electrode 110 configured as a reference electrode, RE, and/or counter electrode, CE, for electrochemical sensing of target analytes, wherein the electrode 110 is disposed on a substrate 105. The device 100 can also include a second microneedle electrode 120 configured as a first working electrode, WE-1, a third microneedle electrode 130 configured as a second working electrode, WE-2, a fourth microneedle electrode 140 configured as a third working electrode, WE-3, and a fifth microneedle electrode 150 configured as a fourth working electrode, WE-4, each disposed on the substrate 105. Each of the microneedle electrodes 110, 120, 130, 140 and 150 of the electrochemical microneedle sensor device 100 can include the microneedle structure and the electrode probe structure, as previously described. The microneedle electrodes 110, 120, 130, 140 and 150 shown in FIG. 6 have microneedle structures 113, 123, 133, 143, and 153, respectively. In various example embodiments, the electrode probe structure can be disposed within the interior or within the opening or cavity of the respective microneedle structure. The microneedle electrodes 110, 120, 130, 140 and 150 shown in FIG. 6 have electrode probe structures 117, 127, 137, 147, and 157, respectively.

In some embodiments, for example, depending on the electrochemical sensing technique applied at the particular working electrode, the first working electrode 120, the second working electrode 130, the third working electrode 140, and the fourth working electrode 150 can include one or more functionalization materials. In FIG. 6, the first working electrode 120 includes a functionalization material 125 disposed on or integrated with at least a portion of the electrode probe structure 127. Similarly, in some embodiments, the second working electrode 130 includes a functionalization material 135 disposed on or integrated with at least a portion of the electrode probe structure 137. Also, in some embodiments, the third working electrode 140 includes a functionalization material 145 disposed on or integrated with at least a portion of the electrode probe structure 147. Similarly, in some embodiments, the fourth working electrode 150 includes a functionalization material 155 disposed on or integrated with at least a portion of the electrode probe structure 157.

The functionalization materials 125, 135, 145, and 155 of the microneedle sensor device 100 depicted in FIG. 6 can include any of the functionalization materials described above or any other functionalization materials suitable for detection of the target analytes, which can provide information pertinent to assessing a certain condition of a body (e.g., diabetes), at any of the functionalized working electrodes 120, 130, 140, and/or 150 using any of the electrochemical measurement techniques mentioned above. For example, the functionalization material 145 of the electrode 140 can enable antibody-based detection of a target analyte (e.g., insulin), and the functionalization material 155 of the electrode 150 can provide an aptamer-based detection of the same target analyte (insulin).

In an example implementation, the functionalization material 145 is configured to facilitate an electrochemical immunoassay (e.g., including label-free immunoassays) to detect a concentration of an analyte in a biofluid. In some embodiments, the electrochemical microneedle sensor device 100 having at least three or four microneedle-based working electrodes for multiplexed sensing of one or more target analytes includes a microneedle electrode 140 in which the functionalized electrode probe structure 147 is disposed in the hollow interior that is formed as a channel spanning between two openings of the microneedle structure 143 of the electrode 140. Within the channel, the electrode probe structure 147 is functionalized with the functionalization material 145 that includes a capture antibody (e.g., anti-analyte capture and/or detection antibody) covalently connected to a self-assembled monolayer (SAM) coupled to the electrode probe structure 147. The channel allows fluid (e.g., ISF) to flow through the channel within the microneedle structure's interior where the functionalized immunoassay electrode contingent is present to facilitate a detectable reaction. In some example implementations, the anti-analyte antibody-functionalization material 145 can interact with the target analyte for detection at the working electrode (e.g., after covalent attachment of the analyte (e.g., insulin) to the capture antibody and/or subsequent binding of a detection antibody). The immunoassay reaction can cause an electrical signal to be detected at the electrode 140.

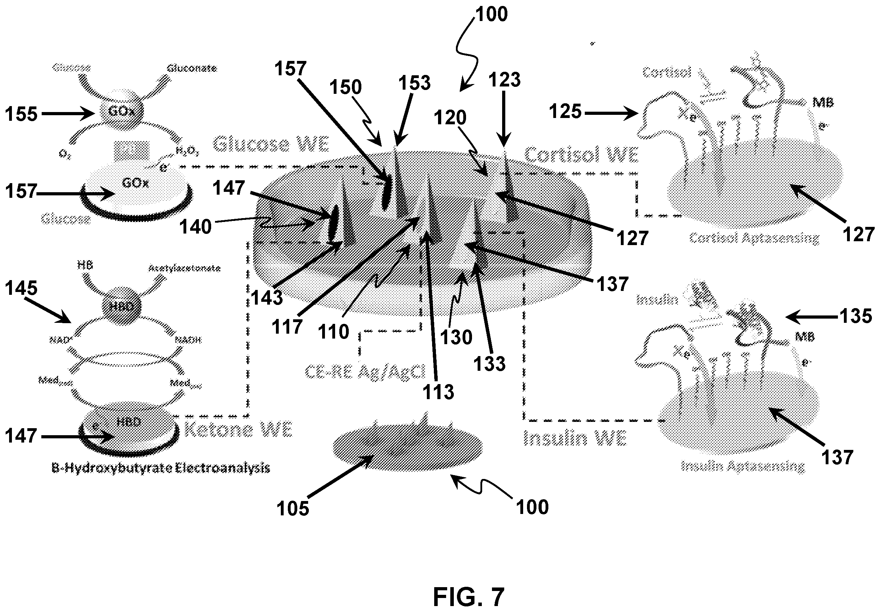

FIG. 7 illustrates an example embodiment of the electrochemical microneedle sensor device 100 having at least four microneedle-based working electrodes for multiplexed sensing of different target analytes in accordance with the technology disclosed in this patent document. The four microneedle-based working electrodes of the device 100 shown in FIG. 7 are disposed on a substrate and are located proximate to a reference and/or counter microneedle-based electrode which is also disposed on the same substrate. As shown in FIG. 7, the device 100 includes a first microneedle electrode 110 configured as a reference electrode, RE, and/or counter electrode, CE, for electrochemical sensing of one or more target analytes, wherein the electrode 110 is disposed on a substrate 105. The device 100 also includes a second microneedle electrode 120 configured as a first working electrode, WE-1, a third microneedle electrode 130 configured as a second working electrode, WE-2, a fourth microneedle electrode 140 configured as a third working electrode, WE-3, and a fifth microneedle electrode 150 configured as a fourth working electrode, WE-4. Each electrode of the microneedle electrodes 120, 130, 140 and 150 is also disposed on the substrate 105. Each of the microneedle electrodes 110, 120, 130, 140 and 150 of the electrochemical microneedle sensor device 100 shown in FIG. 7 includes a microneedle structure and an electrode probe structure. For example, any of the microneedle structures and/or the electrode probe structures can be any of the ones previously described. The microneedle electrodes 110, 120, 130, 140 and 150 shown in FIG. 7 have microneedle structures 113, 123, 133, 143, and 153, respectively. As shown in FIG. 7, the electrode probe structures 117, 127, 137, 147, and 157 of the microneedle electrodes 110, 120, 130, 140 and 150, respectively, are disposed within the interior of the microneedle structure or within an opening or cavity of the microneedle structure 113, 123, 133, 143, and 153, respectively.

In some embodiments, for example, depending on the electrochemical sensing technique applied at the particular working electrode, the first working electrode 120, the second working electrode 130, the third working electrode 140, and the fourth working electrode 150 can include one or more functionalization materials. In FIG. 6, the first working electrode 120 includes a functionalization material 125 disposed on or integrated with at least a portion of the electrode probe structure 127 and/or at least partially disposed within the opening or cavity of the microneedle structure 123. Similarly, in some embodiments, the second working electrode 130 includes a functionalization material 135 disposed on or integrated with at least a portion of the electrode probe structure 137 and/or at least partially disposed within the opening or cavity of the microneedle structure 133. Also, in some embodiments, the third working electrode 140 includes a functionalization material 145 disposed on or integrated with at least a portion of the electrode probe structure 147 and/or at least partially disposed within the opening or cavity of the microneedle structure 143. Similarly, in some embodiments, the fourth working electrode 150 includes a functionalization material 155 disposed on or integrated with at least a portion of the electrode probe structure 157 and/or at least partially disposed within the opening or cavity of the microneedle structure 153.

The functionalization materials 125, 135, 145, and 155 of the microneedle sensor device 100 depicted in FIG. 7 can include any of the functionalization materials described above or any other functionalization materials suitable for detection of the target analytes, that can provide information pertinent a certain condition of a body (e.g., diabetes), at any of the functionalized working electrodes 120, 130, 140, and/or 150 using any of the electrochemical detection techniques mentioned above.

The embodiment of the microneedle sensor device 100 shown in FIG. 7 is configured for real-time measurement of cortisol, insulin, glucose, and ketone bodies all on a single microneedle array patch incorporating the substrate 105. Concentrations of the first two analytes (cortisol, and insulin) are tracked through reversible binding, conformation-dependent aptamers, while concentrations of the latter two analytes, glucose and ketone bodies, are monitored using specific biocatalytic redox reactions of these molecules in the presence of the enzymes, glucose oxidase (GOx) and β-hydroxybutyrate dehydrogenase (HBD), respectively. Such a multiplexed simultaneous monitoring of four important diabetes-related markers can provide a more comprehensive understanding of the patient's state of health toward enhanced glycemic control compared to monitoring of a single marker. Other embodiments of the device 100 can provide measurements of other disease biomarkers, including, for example, such proteins as Interleukin-6 (IL-6), tumor necrosis factor (TNF), C-reactive protein (CRP) etc., in combination with such metabolites, as, for example, alcohol, glutamate, lactic acid etc., as well as in combination with potentiometric-based detection of various electrolytes such as, for example, sodium, potassium, lithium, etc.

The microneedle sensor device 100 shown in FIG. 7 includes four hollow microneedle-based working electrodes 120, 130, 140, and 150. The two Au wire -integrated microneedle electrodes 120 and 130 on the right are functionalized with the cortisol-specific and insulin-specific aptamers, respectively, and the two hollow microneedle electrodes 140 and 150 on the left are packed with carbon paste matrices and act as working electrodes for ketone bodies and glucose, respectively. The Ag/AgCl wire -integrated microneedle-based electrode 110 is the common reference electrode. The electrode 110 is shown in the middle between the working electrodes. Other relative arrangements of the electrodes of the sensor device are possible as well.

The carbon paste-filled microneedle electrode 140 for monitoring concentration of ketone bodies shown in FIG. 7 can be constructed through incorporation of a suitable mediator into the carbon paste, followed by the stable HBD/NAD+ enzyme/cofactor confinement and drop casting of outer polymeric membranes to enable the continuous monitoring of ketone bodies.

Similarly, the carbon paste-filled microneedle electrode 150 shown in FIG 7 and used for monitoring of glucose concentration relies on the immobilized glucose oxidase (GOx) enzyme and reduction of the produced hydrogen peroxide (H₂O₂) on the graphite/mineral oil carbon paste modified with Prussian blue (PB). GOx enzyme and PB can be easily incorporated into the carbon paste without any leaching problem while being used for extended periods of time in the fluid matrix.

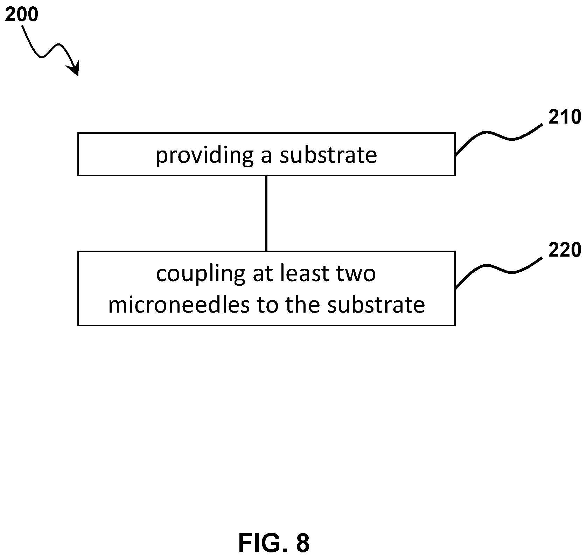

FIG. 8 illustrates a diagram of an example embodiment of a method 200 of manufacturing an electrochemical sensing device. The method 200 includes a step 210 of providing a substrate. The method 200 further includes a step 220 of coupling at least two microneedles to the substrate.

One aspect of the disclosed technology relates to a device that includes a substrate and at least two microneedles coupled to the substrate, wherein each microneedle in the at least two microneedles comprises a protruded needle structure and an electrode probe structure, wherein the protruded needle structure comprises an exterior wall extending outward from a surface of the substrate, the exterior wall circumscribing an interior volume of the protruded needle structure and forming an apex at a terminus point of the exterior wall and wherein the electrode probe structure is configured to produce a signal in response to one or more chemical or biological substances in a biofluid that come in contact with the electrode probe structure, wherein the electrode probe structure of a first microneedle in the at least two microneedles includes an aptamer sequence which is specific for a first analyte, the electrode probe structure of the first microneedle is operable as a working electrode for detection of the first analyte using a first electrochemical detection technique, and wherein the electrode probe structure of a second microneedle in the at least two microneedles is operable as an electrochemical counter electrode or an electrochemical reference electrode.

In some example embodiments, the electrode probe structure is incorporated with or attached to the protruded needle structure. In certain example embodiments, the aptamer sequence is tethered to the electrode probe structure of the first microneedle via a 5'-thiol. In other example embodiments, the aptamer sequence is functionalized with a redox reporter molecule. According to certain example embodiments, the redox reporter molecule is methylene blue. According to other example embodiments, the redox reporter molecule is anthraquinone. In an example embodiment, the functionalization is at a 3' end of the aptamer sequence. In another example embodiment, the functionalization is at a 5' end of the aptamer sequence.

In some example embodiments, the device includes at least two electrically conducting channels, wherein each channel in the at least two electrically conducting channels is electrically coupled to the electrode probe structure of a microneedle in the at least two microneedles to transmit the signal from the electrode probe structure or to apply a control signal to the electrode probe structure. In other example embodiments, the electrode probe structure of a third microneedle in the at least two microneedles includes an aptamer sequence which is specific for a second analyte, the electrode probe structure of the third microneedle is operable as a working electrode for detection of the second analyte using a second electrochemical detection technique. In yet another example embodiment, the second analyte is different from the first analyte. According to an example embodiment, the second electrochemical detection technique is different from the first electrochemical detection technique.

In certain example embodiments, for at least a microneedle in the at least two microneedles, the interior volume of the protruded needle structure of the microneedle comprises a hollow interior defined by an interior wall, the exterior wall of the protruded needle structure of the microneedle comprises an opening to the hollow interior and the electrode probe structure of the microneedle is at least partially disposed within the hollow interior. In yet another example embodiment, the electrode probe structure of a microneedle in the at least two microneedles includes a metal film. In some example embodiments, the electrode probe structure of a microneedle in the at least two microneedles comprises a metal wire. According to certain example embodiments, the metal is gold. In other example embodiments, the electrode probe structure of the second microneedle in the at least two microneedles includes silver/silver chloride (Ag/AgCl).

The electrode probe structure of a fourth microneedle in the at least two microneedles includes a coating on a surface of the electrode probe structure, wherein the coating is functionalized with a first enzyme, wherein the coating is configured to interact with a third analyte, and wherein the electrode probe structure of the fourth microneedle is operable as a working electrode for detection of the third analyte using a third electrochemical detection technique. According to some example embodiments, the electrode probe structure of a fifth microneedle in the at least two microneedles includes a coating on a surface of the electrode probe structure, wherein the coating is functionalized with a second enzyme, wherein the coating is configured to interact with a fourth analyte, and wherein the electrode probe structure of the fifth microneedle is operable as a working electrode for detection of the fourth analyte using a fourth electrochemical detection technique. In certain example embodiments, the second enzyme is different from the first enzyme.

In other example embodiments, the electrode probe structure of a sixth microneedle in the at least two microneedles includes a coating on a surface of the electrode probe structure, wherein the coating is functionalized with an ionophore receptor, wherein the coating is configured to interact with a fifth analyte, wherein the electrode probe structure of the sixth microneedle is operable as a working electrode for detection of the fifth analyte using a fifth electrochemical detection technique, and wherein the fifth analyte is an electrolyte. In some example embodiments, any electrochemical detection technique from the first, second, third, and fourth electrochemical detection techniques is one of: a cyclic voltammetry technique, a fast scan cyclic voltammetry technique, a square wave voltammetry technique, a potentiometric measurement technique, or a chronoamperometry technique. In certain example embodiments, the fifth electrochemical detection techniques is one of: a cyclic voltammetry technique, a fast scan cyclic voltammetry technique, a square wave voltammetry technique, a potentiometric measurement technique, or a chronoamperometry technique.

In other example embodiments, the electrode probe structure of at least one microneedle in the at least two microneedles includes a conformal coating. According to some example embodiments, the conformal coating includes an electrically insulating polymer or a dielectric material. In an example embodiment, the conformal coating includes at least one of: a poly(p-xylylene) polymer, a polyethyleneimine polymer, or SiO2. In other example embodiments, the first analyte is a protein. According to an example embodiment, the protein is a cytokine. In yet another example embodiment, the protein is one of: insulin, an interleukin-6 protein, a tumor necrosis factor protein, or a C-reactive protein. In some example embodiments, the third analyte is a small molecule compound. In other example embodiments, the fourth analyte is a small molecule compound. According to certain example embodiments, the small molecule compound is one of: lactose, lactate, an alcohol, glucose, glutamate, or ketone bodies. In other example embodiments, the first analyte is a drug. In some example embodiments, the first analyte is cortisol and the second analyte is insulin. In other example embodiments, the first enzyme is glucose oxidase and the third analyte is glucose. In yet other example embodiments, the second enzyme is β-hydroxybutyrate dehydrogenase and the fourth analyte is ketone bodies. According to certain example embodiments, the electrolyte is one of: sodium, potassium, or lithium. According to other example embodiments, the third analyte is different from the first analyte and the third analyte is different from the second analyte. In some example embodiments, the fourth analyte is different from the first analyte, the fourth analyte is different from the second analyte, and the fourth analyte is different from the third analyte. In other example embodiments, the device is configured to be disposed on a skin of a person.

Another aspect of the disclosed technology relates to a method of manufacturing an electrochemical sensing device that includes providing a substrate and coupling at least two microneedles to the substrate, wherein each microneedle in the at least two microneedles comprises a protruded needle structure and an electrode probe structure, wherein the protruded needle structure comprises an exterior wall extending outward from a surface of the substrate, the exterior wall circumscribing an interior volume of the protruded needle structure and forming an apex at a terminus point of the exterior wall and wherein the electrode probe structure is configured to produce a signal in response to one or more chemical or biological substances in a biofluid that come in contact with the electrode probe structure, wherein the electrode probe structure of a first microneedle in the at least two microneedles includes an aptamer sequence which is specific for a first analyte, the electrode probe structure of the first microneedle is operable as a working electrode for detection of the first analyte using a first electrochemical detection technique, and wherein the electrode probe structure of a second microneedle in the at least two microneedles is operable as an electrochemical counter electrode or an electrochemical reference electrode.

Yet another aspect of the disclosed technology relates to a method of electrochemical-based sensing that includes providing a device according to the technology disclosed in this patent document, wherein the device comprises at least two electrically conducting channels, wherein each channel in the at least two electrically conducting channels is electrically coupled to the electrode probe structure of a microneedle in the at least two microneedles of the device to transmit a signal (a sensing signal, e.g. a signal which is generated in response to a compound in a biofluid or a tissue) from the electrode probe structure or to apply a control signal to the electrode probe structure. The method further includes transmitting the signal from the electrode probe structure of a first microneedle in the at least two microneedles using a first electrically conducting channel in the at least two electrically conducting channels.

An aspect of the disclosed technology relates to a method of electrochemical-based sensing that includes providing a device according to the technology disclosed in this patent document, wherein the device comprises at least two electrically conducting channels, wherein each channel in the at least two electrically conducting channels is electrically coupled to the electrode probe structure of a microneedle in the at least two microneedles of the device to transmit a signal (e.g., a sensing signal which is, for example, generated in response to a compound in a biofluid or a tissue) from the electrode probe structure or to apply a control signal to the electrode probe structure. The method further includes applying a first control signal to the electrode probe structure of a first microneedle in the at least two microneedles using a first electrically conducting channel in the at least two electrically conducting channels. The method also includes transmitting the signal from the electrode probe structure of a second microneedle in the at least two microneedles using a second electrically conducting channel in the at least two electrically conducting channels. In some implementations, the method of electrochemical-based sensing further includes determining a concentration of an analyte using the signal transmitted from the electrode probe structure of the second microneedle.

In some example embodiments of the method of electrochemical-based sensing, the first microneedle and the second microneedle refer to a same microneedle in the at least two microneedles. In other example embodiments of the method, the first microneedle and the second microneedle refer to different microneedles in the at least two microneedles. In yet other example embodiments of the method, the first electrically conducting channel and the second electrically conducting channel refer to a same electrically conducting channel in the at least two electrically conducting channels. In certain example embodiments of the method, the first electrically conducting channel and the second electrically conducting channel refer to different electrically conducting channels in the at least two electrically conducting channels. According to some example embodiments of the method, an electrically conducting channel in the at least two electrically conducting channels is a wire made of an electrically conducting material or a trace of an electrically conducting material on a circuit board.

It is intended that the specification, together with the drawings, be considered exemplary only, where exemplary means an example. As used herein, the singular forms "a", "an" and "the" are intended to include the plural forms as well, unless the context clearly indicates otherwise. Additionally, the use of "or" is intended to include "and/or", unless the context clearly indicates otherwise.

Certain features that are described in this patent document in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. Moreover, the separation of various system components in the embodiments described in this patent document should not be understood as requiring such separation in all embodiments.

It is understood that the various disclosed embodiments may be implemented individually, or collectively, in devices comprised of various optical components, electronics hardware and/or software modules and components. These devices, for example, may comprise a processor, a memory unit, an interface that are communicatively connected to each other, and may range from desktop and/or laptop computers, to mobile devices and the like. The processor and/or controller can perform various disclosed operations based on execution of program code that is stored on a storage medium. The processor and/or controller can, for example, be in communication with at least one memory and with at least one communication unit that enables the exchange of data and information, directly or indirectly, through the communication link with other entities, devices and networks. The communication unit may provide wired and/or wireless communication capabilities in accordance with one or more communication protocols, and therefore it may comprise the proper transmitter/receiver antennas, circuitry and ports, as well as the encoding/decoding capabilities that may be necessary for proper transmission and/or reception of data and other information. For example, the processor may be configured to receive electrical signals or information from the disclosed sensors (e.g., CMOS sensors), and to process the received information to produce images or other information of interest.

Various information and data processing operations described herein may be implemented in one embodiment by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Therefore, the computer-readable media that is described in the present application comprises non-transitory storage media. Generally, program modules may include routines, programs, objects, components, data structures, etc. that perform particular tasks or implement particular abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes

Only a few implementations and examples are described and other implementations, enhancements and variations can be made based on what is described and illustrated in this patent document.

## Claims

1. A device (100), comprising:
a substrate (105); and
at least three microneedles (110, 120, 130) coupled to the substrate,
wherein each microneedle in the at least three microneedles comprises:
a protruded needle structure (113, 123, 133); and
an electrode probe structure (117, 127, 137),
wherein the protruded needle structure comprises an exterior wall extending outward from a surface of the substrate, the exterior wall circumscribing an interior volume of the protruded needle structure and forming an apex at a terminus point of the exterior wall, and
wherein the electrode probe structure is configured to produce a signal in response to one or more chemical or biological substances in a biofluid that come in contact with the electrode probe structure,
wherein a first electrode probe structure (127) of a first microneedle (120) in the at least three microneedles includes an aptamer sequence which is specific for a first analyte, the aptamer sequence functionalized at a 3' end of the aptamer sequence or at a 5' end of the aptamer sequence with a redox reporter molecule, and wherein the first electrode probe structure is operable as a first working electrode (WE-1) for detection of the first analyte using a first electrochemical detection technique,
wherein a second electrode probe structure (117) of a second microneedle (110) in the at least three microneedles is operable as an electrochemical counter electrode (CE) or an electrochemical reference electrode (RE), and
wherein a third electrode probe structure (137) of a third microneedle (130) in the at least three microneedles is operable as a second working electrode (WE-2) for detection of a second, different analyte using a second, different electrochemical detection technique, and wherein the third electrode probe structure includes a coating on a surface of the third electrode probe structure, the coating functionalized with a first enzyme that is configured to interact with the second analyte.

2. The device of claim 1, wherein the electrode probe structure is incorporated with or attached to the protruded needle structure, or the aptamer sequence is tethered to the electrode probe structure of the first microneedle via a 5'-thiol.

3. The device of claim 1, wherein the redox reporter molecule includes methylene blue or anthraquinone.

4. The device of claim 1, further comprising at least two electrically conducting channels, wherein each channel in the at least two electrically conducting channels is electrically coupled to the electrode probe structure of a microneedle in the at least three microneedles to transmit the signal from the electrode probe structure or to apply a control signal to the electrode probe structure.

5. The device of claim 1, wherein the first enzyme is glucose oxidase and the second analyte is glucose, or wherein the first enzyme is β-hydroxybutyrate dehydrogenase and the second analyte is a ketone body.

6. The device of claim 1, wherein, for at least a microneedle in the at least three microneedles, the interior volume of the protruded needle structure of the microneedle comprises a hollow interior defined by an interior wall, the exterior wall of the protruded needle structure of the microneedle comprises an opening to the hollow interior and the electrode probe structure of the microneedle is at least partially disposed within the hollow interior.

7. The device of claim 1, wherein the electrode probe structure of a microneedle in the at least three microneedles includes a metal film or a metal wire.

8. The device of claim 1, wherein the second electrode probe structure of the second microneedle in the at least three microneedles includes silver/silver chloride (Ag/AgCl).

9. The device of claim 1, wherein the electrode probe structure of at least one microneedle in the at least three microneedles includes a conformal coating that includes an electrically insulating polymer, a dielectric material, a poly(p-xylylene) polymer, a polyethyleneimine polymer, or SiO₂.

10. The device of claim 1, wherein the first analyte is a protein or a drug, wherein optionally, the protein is a cortisol, cytokine, insulin, an interleukin-6 protein, a tumor necrosis factor protein, or a C-reactive protein.

11. The device of claim 1, wherein the device is configured to apply signals to and receive signals from the first working electrode independently from the second working electrode.

12. The device of claim 1, wherein the first electrochemical detection technique is a voltammetry technique, and the second electrochemical detection technique is a chronoamperometry technique.

13. The device of claim 1, wherein the second electrode probe structure is operable as a common electrochemical counter electrode or a common electrochemical reference electrode for the first and second working electrodes.

14. A method (200) of manufacturing an electrochemical sensing device (100), comprising:
providing (210) a substrate (105); and
coupling (220) at least three microneedles (110, 120, 130) to the substrate,
wherein each microneedle in the at least three microneedles comprises:
a protruded needle structure (113, 123, 133); and
an electrode probe structure (117, 127, 137),
wherein the protruded needle structure comprises an exterior wall extending outward from a surface of the substrate, the exterior wall circumscribing an interior volume of the protruded needle structure and forming an apex at a terminus point of the exterior wall, and
wherein the electrode probe structure is configured to produce a signal in response to one or more chemical or biological substances in a biofluid that come in contact with the electrode probe structure,
wherein a first electrode probe structure (127) of a first microneedle (120) in the at least three microneedles includes an aptamer sequence that is specific for a first analyte, the aptamer sequence functionalized at a 3' end of the aptamer sequence or at a 5' end of the aptamer sequence with a redox reporter molecule, and is operable as a first working electrode (WE-1) for detection of the first analyte using a first electrochemical detection technique,
wherein a second electrode probe structure (117) of a second microneedle (110) in the at least three microneedles is operable as an electrochemical counter electrode (CE) or an electrochemical reference electrode (RE), and
wherein a third electrode probe structure (137) of a third microneedle (130) in the at least three microneedles is operable as a second working electrode (WE-2) for detection of a second, different analyte using a second, different electrochemical detection technique, and wherein the third electrode probe structure includes a coating on a surface of the third electrode probe structure, the coating functionalized with a first enzyme that is configured to interact with the second analyte.

## Patentansprüche

1. Vorrichtung (100), umfassend:
ein Substrat (105); und
mindestens drei Mikronadeln (110, 120, 130), die mit dem Substrat verbunden sind,
wobei jede Mikronadel in den mindestens drei Mikronadeln umfasst:
eine hervorstehende Nadelstruktur (113, 123, 133); und
eine Elektrodensondenstruktur (117, 127, 137),
wobei die vorstehende Nadelstruktur eine Außenwand umfasst, die sich von einer Oberfläche des Substrats nach außen erstreckt, wobei die Außenwand ein Innenvolumen der vorstehenden Nadelstruktur abgrenzt und an einem Endpunkt der Außenwand einen Scheitelpunkt bildet, und
wobei die Elektrodensondenstruktur so konfiguriert ist, dass sie ein Signal als Reaktion auf eine oder mehrere chemische oder biologische Substanzen in einer Bioflüssigkeit erzeugt, die mit der Elektrodensondenstruktur in Kontakt kommen,
wobei eine erste Elektrodensondenstruktur (127) einer ersten Mikronadel (120) in den mindestens drei Mikronadeln eine Aptamer-Sequenz einschließt, die für einen ersten Analyten spezifisch ist, wobei die Aptamer-Sequenz an einem 3'-Ende der Aptamer-Sequenz oder an einem 5'-Ende der Aptamer-Sequenz mit einem Redox-Reportermolekül funktionalisiert ist, und wobei die erste Elektrodensondenstruktur als eine erste Arbeitselektrode (WE-1) zum Nachweis des ersten Analyten unter Verwendung einer ersten elektrochemischen Nachweistechnik betreibbar ist,
wobei eine zweite Elektrodensondenstruktur (117) einer zweiten Mikronadel (110) in den mindestens drei Mikronadeln als eine elektrochemische Gegenelektrode (CE) oder eine elektrochemische Referenzelektrode (RE) betreibbar ist, und
wobei eine dritte Elektrodensondenstruktur (137) einer dritten Mikronadel (130) in den mindestens drei Mikronadeln als eine zweite Arbeitselektrode (WE-2) zum Nachweis eines zweiten, unterschiedlichen Analyten unter Verwendung einer zweiten, unterschiedlichen elektrochemischen Nachweistechnik betreibbar ist, und wobei die dritte Elektrodensondenstruktur eine Beschichtung auf einer Oberfläche der dritten Elektrodensondenstruktur einschließt, wobei die Beschichtung mit einem ersten Enzym funktionalisiert ist, das so konfiguriert ist, dass es mit dem zweiten Analyten in Wechselwirkung steht.

2. Vorrichtung nach Anspruch 1, wobei die Elektrodensondenstruktur in die vorstehende Nadelstruktur integriert oder daran befestigt ist, oder die Aptamer-Sequenz über ein 5'-Thiol an die Elektrodensondenstruktur der ersten Mikronadel gebunden ist.

3. Vorrichtung nach Anspruch 1, wobei das Redox-Reportermolekül Methylenblau oder Anthrachinon enthält.

4. Vorrichtung nach Anspruch 1 ferner umfassend mindestens zwei elektrisch leitende Kanäle, wobei jeder Kanal in den mindestens zwei elektrisch leitenden Kanälen elektrisch mit der Elektrodensondenstruktur einer Mikronadel in den mindestens drei Mikronadeln gekoppelt ist, um das Signal von der Elektrodensondenstruktur zu übertragen oder ein Steuersignal an die Elektrodensondenstruktur anzulegen.

5. Vorrichtung nach Anspruch 1, wobei das erste Enzym Glucoseoxidase ist und der zweite Analyt Glucose ist, oder wobei das erste Enzym β-Hydroxybutyrat-Dehydrogenase ist und der zweite Analyt ein Ketonkörper ist.

6. Vorrichtung nach Anspruch 1, wobei für mindestens eine Mikronadel in den mindestens drei Mikronadeln das Innenvolumen der vorstehenden Nadelstruktur der Mikronadel einen hohlen Innenraum umfasst, der durch eine Innenwand definiert ist, die Außenwand der vorstehenden Nadelstruktur der Mikronadel eine Öffnung zu dem hohlen Innenraum umfasst und die Elektrodensondenstruktur der Mikronadel mindestens teilweise innerhalb des hohlen Innenraums angeordnet ist.

7. Vorrichtung nach Anspruch 1, wobei die Elektrodensondenstruktur einer Mikronadel in den mindestens drei Mikronadeln einen Metallfilm oder einen Metalldraht einschließt.

8. Vorrichtung nach Anspruch 1, wobei die zweite Elektrodensondenstruktur der zweiten Mikronadel in den mindestens drei Mikronadeln Silber/Silberchlorid (Ag/AgCl) einschließt.

9. Vorrichtung nach Anspruch 1, wobei die Elektrodensondenstruktur von mindestens einer Mikronadel in den mindestens drei Mikronadeln eine konforme Beschichtung einschließt, die ein elektrisch isolierendes Polymer, ein dielektrisches Material, ein Poly(p-xylylen)-Polymer, ein Polyethylenimin-Polymer oder SiO₂ einschließt.

10. Vorrichtung nach Anspruch 1, wobei es sich bei dem ersten Analyten um ein Protein oder ein Arzneimittel handelt, wobei es sich bei dem Protein optional um ein Cortisol, ein Zytokin, ein Insulin, ein Interleukin-6-Protein, ein Tumor-Nekrose-Faktor-Protein oder ein C-reaktives Protein handelt.

11. Vorrichtung nach Anspruch 1, wobei die Vorrichtung so konfiguriert ist, dass sie unabhängig von der zweiten Arbeitselektrode Signale an die erste Arbeitselektrode anlegt und von dieser Signale empfängt.

12. Vorrichtung nach Anspruch 1, wobei die erste elektrochemische Nachweistechnik eine Voltammetrietechnik und die zweite elektrochemische Nachweistechnik eine Chronoamperometrietechnik ist.

13. Vorrichtung nach Anspruch 1, wobei die zweite Elektrodensondenstruktur als gemeinsame elektrochemische Gegenelektrode oder als gemeinsame elektrochemische Referenzelektrode für die erste und zweite Arbeitselektrode betrieben werden kann.

14. Verfahren (200) zum Herstellen einer elektrochemischen Sensorvorrichtung (100), umfassend:
Bereitstellen (210) eines Substrats (105); und
Koppeln (220) von mindestens drei Mikronadeln (110, 120, 130) mit dem Substrat,
wobei jede Mikronadel in den mindestens drei Mikronadeln umfasst:
eine hervorstehende Nadelstruktur (113, 123, 133); und
eine Elektrodensondenstruktur (117, 127, 137),
wobei die vorstehende Nadelstruktur eine Außenwand umfasst, die sich von einer Oberfläche des Substrats nach außen erstreckt, wobei die Außenwand ein Innenvolumen der vorstehenden Nadelstruktur abgrenzt und an einem Endpunkt der Außenwand einen Scheitelpunkt bildet, und
wobei die Elektrodensondenstruktur so konfiguriert ist, dass sie ein Signal als Reaktion auf eine oder mehrere chemische oder biologische Substanzen in einer Bioflüssigkeit erzeugt, die mit der Elektrodensondenstruktur in Kontakt kommen,
wobei eine erste Elektrodensondenstruktur (127) einer ersten Mikronadel (120) in den mindestens drei Mikronadeln eine Aptamer-Sequenz einschließt, die für einen ersten Analyten spezifisch ist, wobei die Aptamer-Sequenz an einem 3'-Ende der Aptamer-Sequenz oder an einem 5'-Ende der Aptamer-Sequenz mit einem Redox-Reportermolekül funktionalisiert ist und als eine erste Arbeitselektrode (WE-1) zum Nachweis des ersten Analyten unter Verwendung einer ersten elektrochemischen Nachweistechnik betreibbar ist,
wobei eine zweite Elektrodensondenstruktur (117) einer zweiten Mikronadel (110) in den mindestens drei Mikronadeln als eine elektrochemische Gegenelektrode (CE) oder eine elektrochemische Referenzelektrode (RE) betreibbar ist, und
wobei eine dritte Elektrodensondenstruktur (137) einer dritten Mikronadel (130) in den mindestens drei Mikronadeln als eine zweite Arbeitselektrode (WE-2) zum Nachweis eines zweiten, unterschiedlichen Analyten unter Verwendung einer zweiten, unterschiedlichen elektrochemischen Nachweistechnik betreibbar ist, und wobei die dritte Elektrodensondenstruktur eine Beschichtung auf einer Oberfläche der dritten Elektrodensondenstruktur einschließt, wobei die Beschichtung mit einem ersten Enzym funktionalisiert ist, das so konfiguriert ist, dass es mit dem zweiten Analyten in Wechselwirkung steht.

## Revendications

1. Dispositif (100), comprenant :
un substrat (105) ; et
au moins trois micro-aiguilles (110, 120, 130) couplées au substrat,
dans lequel chaque micro-aiguille parmi les au moins trois micro-aiguilles comprend :
une structure d'aiguille saillante (113, 123, 133) ; et
une structure de sonde à électrode (117, 127, 137),
dans lequel la structure d'aiguille saillante comprend une paroi extérieure s'étendant vers l'extérieur à partir d'une surface du substrat, la paroi extérieure circonscrivant un volume intérieur de la structure d'aiguille saillante et formant un sommet au niveau d'un point terminal de la paroi extérieure, et
dans lequel la structure de sonde à électrode est configurée pour produire un signal en réponse à une ou plusieurs substances chimiques ou biologiques présentes dans un biofluide qui entrent en contact avec la structure de sonde à électrode,
dans lequel une première structure de sonde à électrode (127) d'une première micro-aiguille (120) parmi les au moins trois micro-aiguilles comporte une séquence d'aptamère qui est spécifique à un premier analyte, la séquence d'aptamère étant fonctionnalisée à une extrémité 3' de la séquence d'aptamère ou à une extrémité 5' de la séquence d'aptamère avec une molécule rapportrice redox, et dans lequel la première structure de sonde à électrode est utilisable comme première électrode de travail (WE-1) pour la détection du premier analyte à l'aide d'une première technique de détection électrochimique,
dans lequel une deuxième structure de sonde à électrode (117) d'une deuxième micro-aiguille (110) parmi les au moins trois micro-aiguilles est utilisable comme contre-électrode électrochimique (CE) ou électrode de référence électrochimique (RE), et
dans lequel une troisième structure de sonde à électrode (137) d'une troisième micro-aiguille (130) parmi les au moins trois micro-aiguilles est utilisable comme seconde électrode de travail (WE-2) pour la détection d'un second analyte différent à l'aide d'une seconde technique de détection électrochimique différente, et dans lequel la troisième structure de sonde à électrode comporte un revêtement sur une surface de la troisième structure de sonde à électrode, le revêtement étant fonctionnalisé avec une première enzyme qui est configurée pour interagir avec le second analyte.

2. Dispositif selon la revendication 1, dans lequel la structure de sonde à électrode est incorporée à ou attachée à la structure d'aiguille saillante, ou la séquence d'aptamère est liée à la structure de sonde à électrode de la première micro-aiguille via un thiol en 5'.

3. Dispositif selon la revendication 1, dans lequel la molécule rapportrice redox comporte du bleu de méthylène ou de l'anthraquinone.

4. Dispositif selon la revendication 1, comprenant en outre au moins deux canaux conducteurs électriques, dans lequel chaque canal parmi les au moins deux canaux conducteurs électriques est couplé électriquement à la structure de sonde à électrode d'une micro-aiguille parmi les au moins trois micro-aiguilles pour transmettre le signal de la structure de sonde à électrode ou pour appliquer un signal de commande à la structure de sonde à électrode.

5. Dispositif selon la revendication 1, dans lequel la première enzyme est la glucose oxydase et le second analyte est le glucose, ou dans lequel la première enzyme est la β-hydroxybutyrate déshydrogénase et le second analyte est un corps cétonique.

6. Dispositif selon la revendication 1, dans lequel, pour au moins une micro-aiguille parmi les au moins trois micro-aiguilles, le volume intérieur de la structure d'aiguille saillante de la micro-aiguille comprend un intérieur creux défini par une paroi intérieure, la paroi extérieure de la structure d'aiguille saillante de la micro-aiguille comprend une ouverture vers l'intérieur creux et la structure de sonde à électrode de la micro-aiguille est au moins partiellement disposée à l'intérieur de l'intérieur creux.

7. Dispositif selon la revendication 1, dans lequel la structure de sonde à électrode d'une micro-aiguille parmi les au moins trois micro-aiguilles comporte un film métallique ou un fil métallique.

8. Dispositif selon la revendication 1, dans lequel la deuxième structure de sonde à électrode de la deuxième micro-aiguille parmi les au moins trois micro-aiguilles comporte de l'argent/chlorure d'argent (Ag/AgCl).

9. Dispositif selon la revendication 1, dans lequel la structure de sonde à électrode d'au moins une micro-aiguille parmi les au moins trois micro-aiguilles comporte un revêtement conforme qui comporte un polymère électriquement isolant, un matériau diélectrique, un polymère de poly(p-xylylène), un polymère de polyéthylèneimine ou du SiO₂.

10. Dispositif selon la revendication 1, dans lequel le premier analyte est une protéine ou un médicament, dans lequel, éventuellement, la protéine est un cortisol, une cytokine, de l'insuline, une protéine interleukine-6, une protéine facteur de nécrose tumorale ou une protéine C-réactive.

11. Dispositif selon la revendication 1, dans lequel le dispositif est configuré pour appliquer des signaux à la première électrode de travail et recevoir des signaux de celle-ci indépendamment de la seconde électrode de travail.

12. Dispositif selon la revendication 1, dans lequel la première technique de détection électrochimique est une technique de voltampérométrie, et la seconde technique de détection électrochimique est une technique de chronoampérométrie.

13. Dispositif selon la revendication 1, dans lequel la deuxième structure de sonde à électrode est utilisable comme contre-électrode électrochimique commune ou comme électrode de référence électrochimique commune pour les première et seconde électrodes de travail.

14. Procédé (200) de fabrication d'un dispositif de détection électrochimique (100), comprenant :
la fourniture (210) d'un substrat (105) ; et
le couplage (220) d'au moins trois micro-aiguilles (110, 120, 130) au substrat,
dans lequel chaque micro-aiguille parmi les au moins trois micro-aiguilles comprend :
une structure d'aiguille saillante (113, 123, 133) ; et
une structure de sonde à électrode (117, 127, 137),
dans lequel la structure d'aiguille saillante comprend une paroi extérieure s'étendant vers l'extérieur à partir d'une surface du substrat, la paroi extérieure circonscrivant un volume intérieur de la structure d'aiguille saillante et formant un sommet au niveau d'un point terminal de la paroi extérieure, et
dans lequel la structure de sonde à électrode est configurée pour produire un signal en réponse à une ou plusieurs substances chimiques ou biologiques présentes dans un biofluide qui entrent en contact avec la structure de sonde à électrode,
dans lequel une première structure de sonde à électrode (127) d'une première micro-aiguille (120) parmi les au moins trois micro-aiguilles comporte une séquence d'aptamère qui est spécifique à un premier analyte, la séquence d'aptamère étant fonctionnalisée à une extrémité 3' de la séquence d'aptamère ou à une extrémité 5' de la séquence d'aptamère avec une molécule rapportrice redox, et est utilisable comme première électrode de travail (WE-1) pour la détection du premier analyte à l'aide d'une première technique de détection électrochimique,
dans lequel une deuxième structure de sonde à électrode (117) d'une deuxième micro-aiguille (110) parmi les au moins trois micro-aiguilles est utilisable comme contre-électrode électrochimique (CE) ou électrode de référence électrochimique (RE), et
dans lequel une troisième structure de sonde à électrode (137) d'une troisième micro-aiguille (130) parmi les au moins trois micro-aiguilles est utilisable comme seconde électrode de travail (WE-2) pour la détection d'un second analyte différent à l'aide d'une seconde technique de détection électrochimique différente, et dans lequel la troisième structure de sonde à électrode comporte un revêtement sur une surface de la troisième structure de sonde à électrode, le revêtement étant fonctionnalisé avec une première enzyme qui est configurée pour interagir avec le second analyte.
